(19) Europäisches Patentamt
European Patent Office
Office européen des brevets

(11) **EP 3 732 992 A1**

(12) **EUROPÄISCHE PATENTANMELDUNG**

(43) Veröffentlichungstag:
**04.11.2020 Patentblatt 2020/45**

(51) Int Cl.:
*A23L 3/26* *(2006.01)*  *A61L 2/14* *(2006.01)*
*A61L 2/20* *(2006.01)*

(21) Anmeldenummer: **20160321.4**

(22) Anmeldetag: **02.03.2020**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(30) Priorität: **18.03.2019 DE 102019106767**

(71) Anmelder: **Relyon Plasma GmbH**
**93055 Regensburg (DE)**

(72) Erfinder: **NETTESHEIM, Stefan**
**93051 Regensburg (DE)**

(74) Vertreter: **Reichert & Lindner**
**Partnerschaft Patentanwälte**
**Stromerstr. 2A**
**93049 Regensburg (DE)**

(54) **ANORDNUNG ZUR DEKONTAMINATION EINER OBERFLÄCHE VON OBJEKTEN UND VERFAHREN ZUR DEKONTAMINATION EINER OBERFLÄCHE VON OBJEKTEN**

(57) Es ist eine Anordnung und ein Verfahren zur Dekontamination von Oberflächen (2S) von Objekten (2) mit reaktiven Spezies (21) offenbart. In einer Kontaktkammer (10) ist ein Plasmaerzeuger (1) vorgesehen, in dem aktive Spezies (21) erzeugt werden. Über ein Dispensorventil (14) des Plasmaerzeugers (1) werden die aktiven Spezies (21) an die Kontaktkammer (10) abgegeben Die Abgabe erfolgt bei einer vordefinierten Taupunkttemperatur, sodass sich ein gleichmäßiger Kondensatfilm (13) auf der Oberfläche (2S) des Objekts (2) niederschlägt.

Fig. 6

**Beschreibung**

Die vorliegende Erfindung betrifft eine Anordnung zur Dekontamination mindestens einer Oberfläche von mindestens einem Objekt. Die Anordnung umfasst einen Plasmaerzeuger, dem mindestens ein Eingang, ein Ausgang und eine Energiequelle zugeordnet sind.

Ferner betrifft die Erfindung ein Verfahren zur Dekontamination einer Oberfläche von mindestens einem Objekt mit reaktiven Spezies.

Industriell werden Plasmaverfahren derzeit u. a. in der Medizintechnik, der Werkstoffherstellung und der Beleuchtungstechnik eingesetzt. Die Anwendung von Plasma erlaubt prinzipiell eine Reduktion mikrobieller Kontaminanten bei geringen Temperaturen, wobei die Wirkung primär auf den Oberflächen erzielt wird. Erste Versuchsreihen im Labormaßstab zur Plasmaanwendung im Lebensmittelbereich untersuchen hauptsächlich Möglichkeiten zur Inaktivierung unerwünschter Mikroorganismen bei hitzeempfindlichen Lebensmitteln, da herkömmliche thermische Dekontaminationsverfahren bei Produkten, wie beispielsweise frischem Obst und Gemüse, Fleisch oder Eiern, nicht oder nur begrenzt einsetzbar sind. Die Plasmaanwendung gilt auch als potenzielle Alternative zu anderen chemischen (z. B. Chloreinsatz) oder physikalischen (z. B. Hochdruck-, Hochspannungsimpulse, ionisierende Bestrahlung) Verfahren. Als Vorteile des Plasmaverfahrens gelten u. a. eine hohe Wirksamkeit bei niedrigen Temperaturen (i. d. R. < 70 °C); gezielte und verbrauchsgerechte Bereitstellung; geringe Beeinflussung der inneren Produktmatrix; wasser- bzw. lösemittel- und rückstandsfreie sowie ressourceneffiziente Anwendung. Andere Verfahren, wie Hochdruck und/oder ionisierende Strahlung sind komplex oder kostspielig. Die Dekontamination mit UV-Licht ist oft nicht wirksam und durch Schatteneffekte eingeschränkt.

Die deutsche Patentanmeldung DE 10 2008 037 898 A1 betrifft ein Verfahren sowie eine Vorrichtung zur Desinfektion oder Sterilisation von Verpackungsmaterial und/oder Behältern und/oder Filtermaterial, wobei das Material bzw. der Behälter mit einem in einem Plasmareaktor erzeugten Gas behandelt wird. Durch die Plasmabehandlung wird eine deutlich verbesserte Entkeimung von entsprechenden Materialien, insbesondere Verpackungsmaterialien und/oder Behältern zur Verpackung und/oder Lagerung von Lebensmitteln, Getränken und anderen hygienisch sensiblen Substanzen, erreicht. Schließlich wird noch die Verwendung von Plasma zur Behandlung solcher Materialien oder Behälter sowie entsprechend behandelte Materialien oder Behälter vorgeschlagen.

Die deutsche Patentanmeldung DE 100 36 550 A1 betrifft ein Sterilisationsverfahren, bei dem die zu behandelnde Oberfläche einer Gasentladung ausgesetzt wird und bei dem die Sterilisation in einer Wasserstoff- und sauerstoffhaltigen Gasatmosphäre bei einem Druck von 10-4·Pa bis 2 x10-5·Pa erfolgt. Das Verfahren lässt sich zur Sterilisation von Verpackungsmaterialien, z. B. Lebensmittelverpackungen oder Etiketten, aber auch für die Sterilisation von Oberflächen für medizinische und/oder biologische und/oder biotechnologische Anwendungen verwenden.

Die deutsche Patentanmeldung DE 10 2015 119 369 A1 betrifft eine Vorrichtung sowie ein System und ein Verfahren zur Behandlung eines Gegenstandes, insbesondere eines oder mehrerer Freiformkörper, mit Plasma. Die Vorrichtung dient zur Behandlung eines Gegenstandes mit Plasma und umfasst eine Hülleinrichtung, mit der ein im Wesentlichen gasdichter Aufnahmeraum ausgebildet ist oder ausbildbar ist, in dem ein zu behandelnder Gegenstand aufnehmbar ist. Des Weiteren umfasst die Vorrichtung eine erste Elektrode sowie eine zweite Elektrode, wobei die beiden Elektroden in Bezug zur Hülleinrichtung derart angeordnet sind, dass bei Anlage einer elektrischen Potenzialdifferenz an den Elektroden ein Plasma im Aufnahmeraum der Hülleinrichtung erzeugbar ist.

Die europäische Patentanmeldung EP 3 085 244 A1 offenbart einen nichtthermischen Plasmareaktor zur Sterilisation von organischen Produkten.

Die US-Patentanmeldung US 2017/112157 A1 offenbart ein Verfahren zur Behandlung einer Oberfläche mit einem reaktiven Gas. Das reaktive Gas wird aus kalten Plasma bei Hochspannung aus einem Arbeitsgas hergestellt (HVCP).

Die deutsche Patentanmeldung DE 10 2014 213 799 A1 offenbart ein Haushaltskältegerät mit einer Lebensmittel-Behandlungseinheit sowie Verfahren zum Betreiben eines derartigen Haushaltskältegeräts. Das Haushaltskältegerät ist mit einem Innenraum zur Aufnahme von Lebensmitteln versehen, der durch Wände eines Innenbehälters begrenzt ist. Ferner ist eine Lebensmittel-Behandlungseinheit vorgesehen, wobei die Lebensmittel-Behandlungseinheit zur Einwirkung auf eine Oberfläche, von in dem Lagerbereich eingebrachte Lebensmittel, in dem Haushaltskältegerät angeordnet und so ausgebildet, dass das Einwirken eine Dekontamination von Pestiziden und/oder Schwermetallen im Lebensmittel ist.

Die deutsche Patentanmeldung DE 10 2005 061 247 A1 offenbart ein Verfahren und eine Vorrichtung zum Entkeimen von Lebensmitteln. Das Lebensmittel wird mit mindestens einem atmosphärischen Plasmastrahl beaufschlagt. Durch die im Plasmastrahl enthaltene Energie wird die Entkeimung der Oberfläche des Lebensmittels durchgeführt.

Die deutsche Patentanmeldung DE 10 2009 025 864 A1 offenbart ein Verfahren zur Entkeimung von Produkten, die als Feststoffe vorliegenden. Das zu entkeimende Produkt wird zunächst entstaubt, dann einem kalten Plasma ausgesetzt und während der Plasmabehandlung gewendet. Ebenso ist eine Vorrichtung zur Durchführung dieses Verfahrens offenbart.

Die deutsche Patentanmeldung DE 10 2015 204 753 A1 betrifft ein Verfahren zum Verkleben einer Substratoberfläche eines Substrates mit einer Klebemasseoberfläche einer Klebemasse. Es wird ein Niedertemperatur-Plasma in einem Niedertemperatur-Plasmagenerator erzeugt. Die Substratoberfläche und/oder die Klebemasseoberfläche werden mit dem Niedertemperatur-Plasma aktiviert. Danach werden zur Ausbildung eines Verklebungsverbundes die Substratoberfläche und die Klebemasseoberfläche aufeinandergeschichtet.

Der Erfindung liegt die Aufgabe zugrunde, eine Anordnung zur Dekontamination von Oberflächen von mindestens einem Objekt bereitzustellen, wobei die thermische Belastung der Objekte gering bzw. vernachlässigbar ist und dennoch die gesamte Oberfläche eines jeden Objekts zur Dekontamination erreicht wird.

Die obige Aufgabe wird durch eine Anordnung zur Dekontamination einer Oberfläche von mindestens einem Objekt gelöst, das die Merkmale des Anspruchs 1 umfasst.

Eine weitere Aufgabe der Erfindung ist ein automatisches Verfahren zur Dekontamination von Oberflächen von mindestens einem Objekt bereitzustellen, wobei die thermische Belastung der Objekte gering bzw. vernachlässigbar ist und dennoch die gesamte Oberfläche eines jeden Objekts zur Dekontamination erreicht wird.

Die obige Aufgabe wird durch ein Verfahren zur Dekontamination von Oberflächen von Objekten mit reaktiven Spezies gelöst, das die Merkmale des Anspruchs 6 umfasst.

Erfindungsgemäß umfasst die Anordnung zur Dekontamination einer Oberfläche von mindestens einem Objekt einen Plasmaerzeuger, dem mindestens ein Eingang, ein Ausgang und eine Energiequelle zugeordnet ist. Eine Kontaktkammer der Anordnung ist zumindest mit einer Dosiereinheit des Plasmaerzeugers fluide verbunden. Mit der Dosiereinheit kann je nach Art der Behandlung des Objekts ein im Plasmaerzeuger erzeugtes Plasma, eine Strahlung, langlebige Radikale und metastabile sowie inhibitorische Stoffe oder ausschließlich reaktive Spezies sein. Eine andere Dosiereinheit ist mit dem Plasmaerzeuger über den mindestens einen Eingang fluide verbunden. Dem Plasmaerzeuger können somit Substanzen zugeführt werden, die zur Erzeugung von reaktiven Spezies oder langlebigen Radikalen und metastabilen Stoffen dienen. Eine Auflage zur Aufnahme des zu behandelnden Objekts ist in der Kontaktkammer vorgesehen. Die Auflage ist gegenüber der Dosiereinheit angeordnet, sodass die aus der Dosiereinheit gesteuert austretenden reaktiven Spezies einen gleichmäßigen Kondensatfilm auf der Oberfläche des Objekts ausbilden können.

Die aus der Dosiereinheit in die Kontaktkammer eintretenden reaktiven Spezies weisen eine Taupunkttemperatur auf, die größer ist als eine Objekttemperatur. Dem Plasmaerzeuger ist eine Energiequelle zugeordnet, mit die Temperatur im Plasmaerzeuger eingestellt werden kann. Die Energiequelle und die Dosiereinheit sind mit einer Steuerung der Anordnung verbunden, so dass damit die Taupunkttemperatur der reaktiven Spezies wunschgemäß eingestellt werden kann.

Damit eine gleichmäßige Kondensation der reaktiven Spezies auch der Oberfläche des Objekts möglich ist, kann die Auflage eine Bewegungseinrichtung zur Aufnahme des Objekts in der Kontaktkammer sein. Die Bewegungseinrichtung bewegt das Objekt derart, dass der gleichmäßige Kondensatfilm aus den reaktiven Spezies auf der Oberfläche des Objekts ausbildbar ist.

Ein Abstand zwischen dem auf der Auflage oder auf der Bewegungseinrichtung liegenden Objekt und der Dosiereinheit kann einstellt werden. Damit kann das auf der Auflage positionierte und zu behandelnde Objekt in Bezug auf die Dosiereinheit positioniert werden. Dadurch kann man verschiedene Behandlungsmodi für das Objekt einstellen.

Gemäß einer möglichen Ausführungsform kann der Plasmaerzeuger außerhalb der Kontaktkammer vorgesehen sein.

Das erfindungsgemäße Verfahren zur Dekontamination von Oberflächen von Objekten mit reaktiven Spezies zeichnet sich dadurch aus, dass zunächst in einer Kontaktkammer mindestens ein Objekt auf einer Auflage positioniert wird. Danach werden einem Plasmareaktor von einer Dosiereinheit für die Behandlung des Objekts ausgewählte Substanzen zugeführt, wobei im Plasmareaktor daraus reaktive Spezies gebildet werden. Über eine weitere, dem Plasmareaktor zugeordnete, Dosiereinheit können die reaktiven Spezies in die Kontaktkammer verbracht werden, wobei über die Dosiereinheit eine Eingangsmischung und die Taupunkttemperatur eingestellt werden. Es ist eine Steuerung vorgesehen, die mit dem Plasmareaktor, einer Energiequelle für den Plasmareaktor und auch der Dosiereinheit verbunden ist. Dadurch kann die Taupunkttemperatur der reaktiven Spezies am Übergang der Dosiereinheit in die Kontaktkammer derart eingestellt werden, dass die Taupunkttemperatur größer als eine Temperatur des Objekts ist, so dass ein gleichmäßiger Kondensatfilm auf der Oberfläche des Objekts ausgebildet wird.

Das erfindungsgemäße Verfahren hat den Vorteil, dass durch den gleichmäßigen Niederschlag des Kondensatfilms, auf der Oberfläche des Objekts, eine wirkungsvolle und, im Wesentlichen, sichere Dekontamination in allen Bereichen der Oberfläche des Objekts erreicht wird.

Die Dekontamination wird durch eine Bewegungseinrichtung unterstützt, mit der das mindestens eine zu behandelnde Objekt während der Behandlungsdauer mit den reaktiven Spezies bewegt wird. Die Bewegung des Objekts unterstützt die Ausbildung eines gleichmäßigen Kondensatfilms der reaktiven Spezies auf der Oberfläche des Objekts und parallel dazu, werden alle Oberflächenbereiche des Objekts erreicht.

Dem Plasmaerzeuger ist eine Energiequelle zugeordnet, mit der eine Stabilisierung der Temperatur im Plasmaerzeuger durch Zuheizen oder Kühlen erzielt wird. Dies ist ebenfalls förderlich für die Ausbildung des gleichmäßigen Kondensatfilms, da die Taupunkttemperatur je nach Erfordernis eingestellt werden kann.

Nach der Behandlung des Objekts mit den reaktiven Spezies kann das Objekt zur Trocknung in eine Türschleuse verbracht werden. Die Schleuse ist mit einer weiteren Heizung/Kühlung versehen, mit der die Temperatur für die Trocknung in der Türschleuse eingestellt werden kann. Dadurch kann der Trocknungsprozess optimal an die Gegebenheiten des Objekts angepasst werden.

Zur Steuerung und Regelung der Dekontamination ist eine Steuereinheit mit mehreren Elementen für die Einstellung und/oder Einhaltung der Bedingungen in der Kontaktkammer kommunikativ verbunden. Die Elemente umfassen, ohne darauf beschränkt zu sein, die Dosiereinheit, das Zirkulationsgebläse, die weitere Dosiereinheit, den Plasmaerzeuger, die Energiequelle des Plasmaerzeugers, mindestens einen Sensor, die Antriebseinheit für die Bewegungseinheit, die Heizung/Kühlung, die Filtereinheit sowie die Türschleuse.

**Definition**

Dekontamination im Zusammenhang mit der vorliegenden Erfindung ist das Entfernen von gefährlichen oder schädlichen Verunreinigungen (Kontaminationen) von Personen, Objekten, wie z. B. Lebensmitteln, Kleidungsstücken, Pflanzen, Tieren, Böden, Festkörpern, Flüssigkeiten.

Die schädlichen Verunreinigungen können dabei chemischer oder biologischer Natur sein. Besonders hervorzuheben sind: giftige, organische Verbindungen, Mikroorganismen aller Art (Viren, Bakterien, Pilze, Sporen, primitive Parasiten).

Im Folgenden sollen Ausführungsbeispiele die Erfindung und ihre Vorteile anhand der beigefügten Figuren näher erläutern. Die Größenverhältnisse in den Figuren entsprechen nicht immer den realen Größenverhältnissen, da einige Formen vereinfacht und andere Formen zur besseren Veranschaulichung vergrößert im Verhältnis zu anderen Elementen dargestellt sind. Dabei zeigen:

**Figur 1**  eine schematische Darstellung einer möglichen Auswahl von Substanzen (organische Substanzen), die auf der Oberfläche des Objekts vorhanden sein können;

**Figur 2**  eine schematische Darstellung der Behandlung eines Objekts mit einem Plasma und dessen Überführung an die Umgebung;

**Figur 3**  eine schematische Darstellung einer Ausführungsform der erfindungsgemäßen Anordnung zum Behandeln von Objekten mit einem Plasma bzw. reaktiven Spezies;

**Figur 4**  eine schematische Darstellung eines Objekts mit einem aus der Oberfläche kondensierten Kondensatfilm;

**Figur 5**  eine beispielhafte Darstellung des Plasmareaktors und der dazu erforderlichen Parameter zum Betrieb desselben; und

**Figur 6**  eine schematische Darstellung einer möglichen Ausführungsform der Anordnung zur Behandlung von Objekten mit Plasma bzw. reaktiven Spezien.

Für gleiche oder gleich wirkende Elemente der Erfindung werden identische Bezugszeichen verwendet. Ferner werden der Übersicht halber nur Bezugszeichen in den einzelnen Figuren dargestellt, die für die Beschreibung der jeweiligen Figuren erforderlich sind.

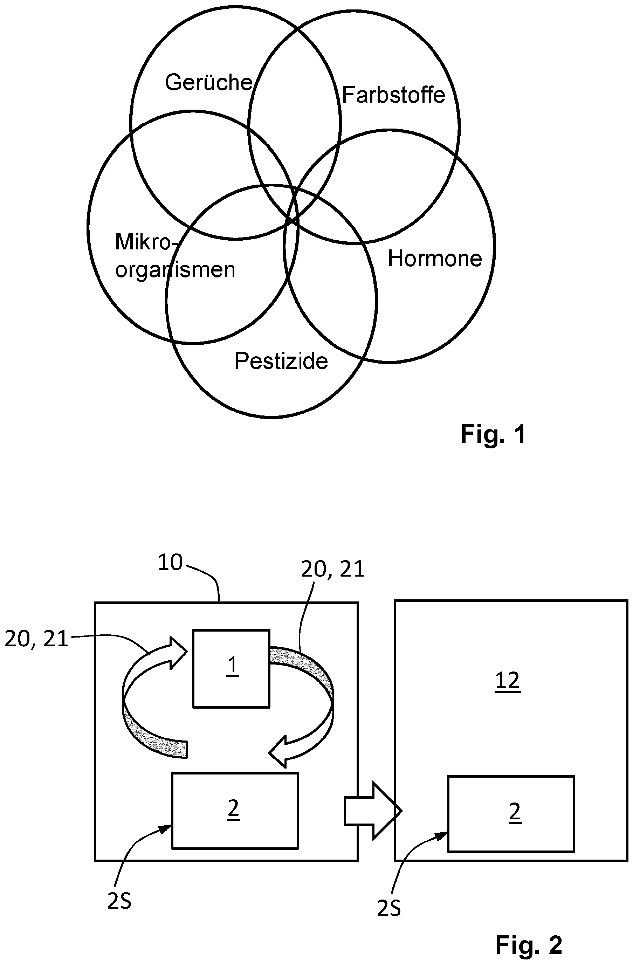

**Figur 1** zeigt eine schematische Darstellung der Substanzen (organische Substanzen), die auf dem Objekt (hier nicht dargestellt) vorhanden sein können. Die organischen Substanzen umfassen, sind jedoch nicht darauf beschränkt, Gerüche (Geruchsmoleküle), Farbstoffe, Hormone, Pestizide oder Mikroorganismen.

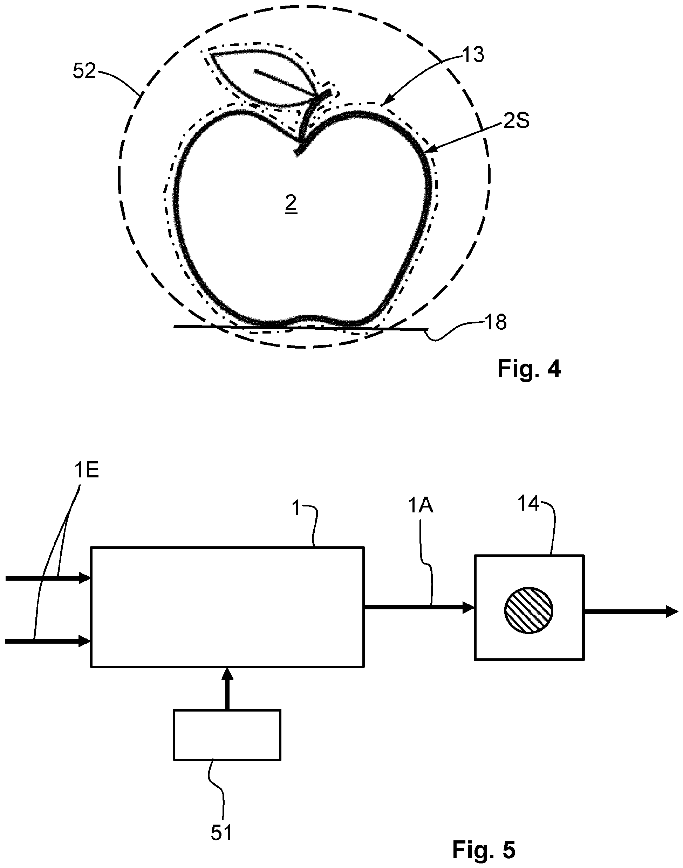

**Figur 2** zeigt eine schematische Darstellung der Behandlung eines Objekts 2 mit einem Plasma 20 bzw. reaktiven Spezies 21, um eine Dekontamination der Oberfläche des Objekts 2S (siehe Figur 4) zu erzielen. Das Plasma 20 bzw. die reaktive Spezies 21 werden unter Verwendung eines Plasmaerzeugers 1 erzeugt. Das bei der Behandlung in der Kammer 10 verbleibende Plasma 20 bzw. die reaktive Spezies 21 werden von dem Plasmaerzeuger 1 erneut zur Erzeugung von Plasma 20 bzw. von reaktiven Spezies 21 verwendet. Nach der Behandlung (Einwirkzeit) mit dem Plasma 20 bzw. den reaktiven Spezies 21 kann das Objekt 2 der normalen Umgebung 12 ausgesetzt werden. Es ist von Vorteil, wenn das Objekt 2 mit sehr feuchtem Plasma 20 bzw. den reaktiven Spezies 21 behandelt wird. Das feuchte Plasma 20 bzw. die reaktiven Spezies 21 beschleunigen und verbessern die Behandlung, da bei geeigneter Einstellung der Parameter der Anordnung, das feuchte Plasma 20 bzw. die reaktiven Spezies 21 auf einer Oberfläche 2S des Objekts 2 kondensieren und somit alle Bereiche der Oberfläche 2S des Objekts 2 erreicht werden. Die Behandlung betrifft im Wesentlichen eine Oberfläche 2S von mindestens einem Objekt 2. Die Verbesserrungen oder Beschleunigungen werden bei der Behandlung von Lebensmitteln, beim Abbau des Pilzbefalls an der Oberfläche, beim Pestizidabbau, bei der Reduzierung von Gerüchen, beim Bleichen organischer Farben oder bei der Entfernung von Bakterien von der Verpackung von Lebensmitteln erreicht. Die nachstehende Tabelle zeigt das Oxidationspotential der verschiedenen im feuchten Plasma entstehenden Moleküle oder Radikale:

| Oxidationspotential [eV] (Standardoxiddationspotential) | | |
|---|---|---|
| $F_2$ | Fluor | 3,06 |
| *OH | Hydroxylradikal | 2,8 |
| *O | Atomarer Sauerstoff | 2,42 |
| $O_3$ | Ozon | 2,08 |
| $H_2O_2$ | Wasserstoffperoxid | 1,77 |
| HClO | Hypochlorige Säure | 1,49 |
| $Cl_2$ | Clor | 1,36 |
| $ClO_2$ | Clordioxid | 1,27 |
| $O_2$ | Sauerstoff | 1,23 |

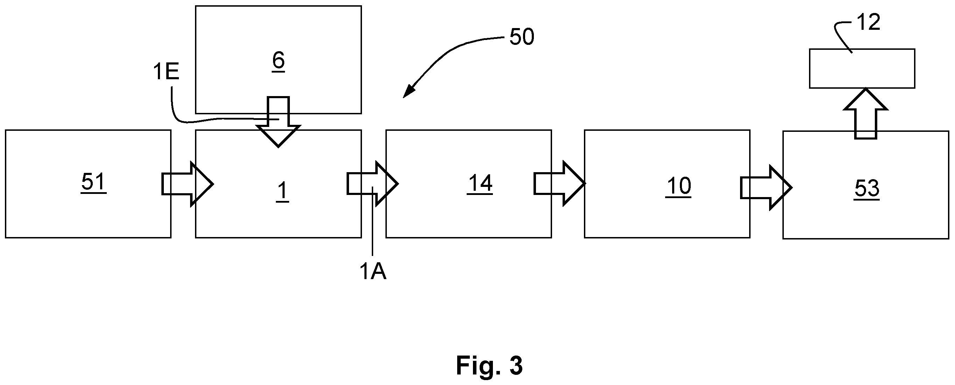

**Figur 3** zeigt eine schematische Darstellung einer Ausführungsform der erfindungsgemäßen Anordnung 50 zum Behandeln von Objekten 2 mit einem Plasma 20 bzw. reaktiven Spezies 21. Die Anordnung 50 umfasst den Plasmaerzeuger 1, dem eine Energiequelle 51 zugeordnet ist. Die Energiequelle 51 kann eine Wärmequelle, eine Wärmesenke, ein elektrisches Potential oder Licht ($h\upsilon$) umfassen. Die Energiequelle 51 wirkt in geeigneter Weise auf den Plasmaerzeuger 1 bzw. Plasmareaktor ein, um ein Plasma gemäß den vorgegebenen Parametern zu erzeugen und den erforderlichen Taupunkt bzw. die Taupunkttemperatur einzustellen.

Der Plasmaerzeuger 1 hat mindestens einen Eingang 1E ausgebildet, über den eine Dosiereinheit 6 mit dem Plasmaerzeuger 1 verbunden ist. Über die Dosiereinheit 6 können dem Plasmaerzeuger 1 Zusatzstoffe, wie z. B. $H_2O$, $O_2$, $N_2$, Spurengas, Salze, Säuren und/oder organische Verbindungen, zugeführt werden. Die Dosiereinheit 6 dient zur Einstellung der Eingangsmischung für den Plasmaerzeuger 1 und des Taupunktes des gebildeten Plasmas 20 bzw. der reaktiven Spezies 21.

Der Plasmaerzeuger 1 besitzt einen Ausgang 1A, über den das Plasma 20 aus dem Plasmaerzeuger 1 zu einer weiteren Dosiereinheit 14 gelangt, die als Dosierventil, Dispenserventil, Dispenserdüse, Öffnung oder Auslass ausgebildet sein kann. Über das Dispenserventil 14 werden der Kontaktkammer 10 bzw. einem Kontaktbereich 52 die angeregten Zusatzstoffe, wie z.B. $H_2O$, $O_2$, $N_2$, $O_3$, $H_2O_2$, OH, $HNO_3$, $HNO_2$, NOX und/oder organische Verbindungen zugeführt.

Für die Behandlung des Objekts 2 in der Kontaktkammer 10 bzw. im Kontaktbereich 52 werden Parameter, wie z. B. Zeit (Verweildauer des Objekts 2 in der Kontaktkammer 10 bzw. im Kontaktbereich 52), Temperatur (Temperatur in der Kontaktkammer 10 bzw. im Kontaktbereich 52), Dosis der auf das Objekt 2 einwirkenden, angeregten Zusatzstoffe, pH-Wert und/oder der Taupunkt (Temperatur an der Oberfläche 2S des Objekts 2) derart eingestellt, dass der Taupunkt an der Oberfläche 2S des Objekts 2 unterschritten wird. Durch die Unterschreitung des Taupunktes an der Oberfläche 2S des Objekts 2 bildet sich ein gleichmäßiger Kondensatfilm 13 auf der Oberfläche 2S des Objekts 2 aus. Dadurch ist sichergestellt, dass alle Bereiche der Oberfläche 2S des Objekts 2 von dem Plasma 20 bzw. der reaktiven Spezies 21 erreicht werden, woraus eine wirkungsvolle Dekontamination der Oberfläche 2S des Objekts 2 erreicht werden kann (nicht dargestellt).

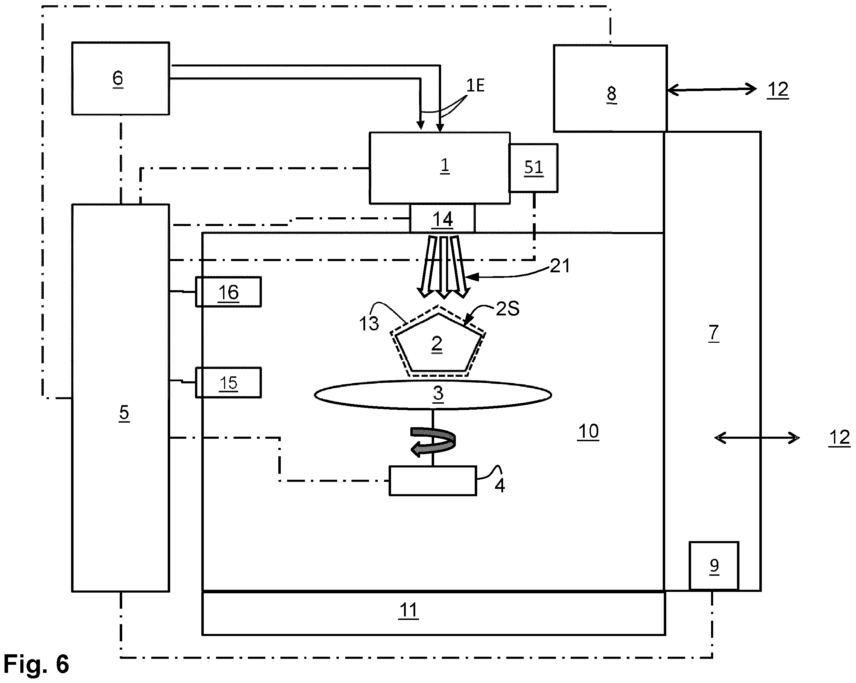

Nach der Behandlung des Objekts 2 mit dem Plasma 20 bzw. den reaktiven Spezies 21 wird das Objekt 2 über eine Entnahmeschleuse 53 aus der Kontaktkammer 10 bzw. dem Kontaktbereich 52 entnommen. Das entsprechend behandelte Objekt 2 kann der regulären Umgebung 12 zugeführt werden. In der Entnahmeschleuse 53 können Parameter, wie z.B. Abbauzeit der reaktiven Spezies 21, Belüftung und/oder Trocknung des Objekts 2 eingestellt werden. Der Kontaktbereich 52, in dem das Plasma 20 bzw. die reaktiven Spezies 21 auf der Oberfläche 2S des Objekts 2 kondensiert, kann als für sich abgeschlossener Bereich in Form der Kontaktkammer 10 ausgebildet sein. Ebenso ist es denkbar, dass das zu behandelnde Objekt 2 der weiteren Dosiereinheit 14 am Ausgang 1A des Plasmaerzeugers 1 vorgesehen ist. Bei dieser möglichen Ausführungsform befinden sich der Plasmaerzeuger 1 und das zu behandelnde Objekt 2 in einer gemeinsamen Kontaktkammer 10 (siehe Figur 6). Das Plasma 20 bzw. die reaktiven Spezies 21 gelangt direkt von der weiteren Dosiereinheit 14 am Ausgang 1A des Plasmaerzeugers 1 zum Kontaktbereich 52 um das Objekt 2 zu kondensieren.

In **Figur 4** ist beispielsweise ein Objekt 2 dargestellt, dessen Oberfläche 2S mit dem Plasma 20 bzw. den reaktiven Spezies 21 behandelt werden soll. Das Objekt 2 ist ein Apfel, was aber nicht als eine Beschränkung der Erfindung aufgefasst werden soll. Wie bereits eingangs erwähnt, kann das Objekt 2 eine Vielzahl von Gegenständen sein, die regelmäßige oder unregelmäßig geformte Oberflächen 2S besitzen. Damit sich das feuchte Plasma 20 mit der Vielzahl an reaktiven Spezies 21 auf der Oberfläche 2S des Objekts 2 niederschlagen kann, muss der Taupunkt bzw. die Taupunkttemperatur des feuchten Plasmas 20 mit den reaktiven Spezies 21 eingestellt werden. Ebenso ist es von Vorteil, wenn das Objekt 2 auf einer Auflage 18 mit Durchbrechungen (nicht dargestellt) oder auf der Auflage 18 mit möglichst wenigen Unterstützungspunkten (nicht dargestellt) gehalten wird, damit sind das feuchte Plasma 20 mit den reaktiven Spezies weitestgehend auf der gesamten Oberfläche 2S des Objekts 2 niederschlagen kann.

Ferner ist es denkbar, dass das Objekt 2 während der Behandlung mit dem feuchten Plasma 20 mit den reaktiven Spezies 21 in sich gedreht wird, damit jeder Abschnitt der Oberfläche 2S des Objekts dem feuchten Plasma 20 mit den reaktiven Spezies 21 ausgesetzt wird.

**Figur 5** zeigt eine beispielhafte Darstellung des Plasmaerzeugers 1 (Plasmareaktor), in dem das feuchte Plasma 20 mit den reaktiven Spezies 21 erzeugt wird. Um das feuchte Plasma 20 mit den reaktiven Spezies 21 bei einer vorgegebenen Taupunkttemperatur und der gewünschten Zusammensetzung zu erhalten, müssen eine Reihe von Parametern eingehalten werden. Dem Plasmaerzeuger 1 wird z. B. Luft mit einer vorbestimmten Rate über einen Eingang 1E zugeführt (die Rate kann z. B. von 0 - 1 l/min betragen). Ebenso kann dem Plasmaerzeuger 1 über einen weiteren oder dem gleichen Eingang 1E Wasser mit einer vorbestimmten Rate zugeführt werden (die Rate kann z. B. 0 - 1 g/min betragen). Es ist für einen Fachmann selbstverständlich, dass die angegebenen Bereiche lediglich ein Beispiel darstellen, was nicht als eine Beschränkung der Erfindung aufgefasst werden soll. Falls dem Plasmaerzeuger 1 beispielsweise eine Leistung zur Erzeugung des feuchten Plasmas 20 mit den reaktiven Spezies 21 zugeführt wird, setzt sich diese aus einer thermischen Leistung $dQ_{th}$ und einer elektrischen Leistung $dQ_e$ zusammen.

Das nachstehende Beispiel zeigt, dass die Leistung, die für eine Ausgangstemperatur $T_{out}$ von 100°C des Plasmas 20 mit den reaktiven Spezies 21 bei einem 1:1-Verhältnis zwischen Wasser und Luft benötigt wird, 100W beträgt.

Für Luft gilt:

$$1,18g/60s * 2J/(g*K) * 80K = 3,14 \text{ W}$$

Für Wasser (l):

$$1g/60s * 4,18J//g*K) = 5,57 \text{ W}$$

Für Wasser (g):

$$1g/60s * 2260J/g = 37,7 \text{ W}$$

Die Gesamtleistung $P_{total}$ = 46 W, so dass die Verlustleistung $P_{loss}$ = 54 W beträgt.

Aus dem Plasmaerzeuger 1 wird das feuchte Plasma 20 mit den reaktiven Spezies 21 über die weitere Dosiereinheit 14 der Kontaktkammer 10 (siehe Figur 3) oder einem Kontaktbereich 52 (siehe Figur 6) zur Behandlung des Objekts 2 mit dem feuchten Plasma 20 und den reaktiven Spezies 21 zugeführt.

Das Plasma 20 alleine und/oder das Plasma 20 mit den reaktiven Spezies 21 kann je nach Zusammensetzung und der direkten, semi-direkten oder indirekten Wirkung auf verschiedene Weise das Objekt 2 beeinflussen.

Bei der direkten Anwendung ist das Plasma 20 in direkten Kontakt mit dem Objekt 2. Die Wechselwirkung mit Objekt 2 basiert auf Strahlung (VUV, UV), geladenen Molekülen, Radikalen und reaktiven Teilchen. Beispiele für die direkte Anwendung sind z. B., ein Jet-Plasma oder ein Plasma, das mittels dielektrischer Barrierenentladung erzeugt wird.

Bei der semi-direkten Anwendung ist der Abstand A zwischen dem Austritt des Plasmas am Plasmaerzeuger 1 und dem Objekt 2 derart bemessen, dass dieser größer als die mittlere freie Weglänge ist. Es liegt somit keine direkte Wechselwirkung zwischen den geladenen Teilchen des Plasmas und dem Objekt 2 vor. Der antimikrobielle Effekt wird durch Strahlung, langlebige Radikale und metastabile sowie inhibitorische Stoffe bewirkt. Beispiele für die semi-direkte Anwendung ist eine semidielektrische Barrierenentladung mit einem Abstand A zum Objekt 2 oder das Sterrad-Verfahren, welcher die Sterilisation bei Niedertemperatur mit Wasserstoffperoxid betrifft (nicht dargestellt).

Bei der indirekten Methode gibt es zwei mögliche Verfahren. Beim ersten Verfahren erfolgt die Bestrahlung des Objekts 2 mit UV- oder VUV-Licht. Das Plasma ist dabei im UV oder VUV transparenten Reaktor eingeschlossen. Das Objekt 2 hat folglich keine Interaktion mit geladenen Teilchen. Das in UV-Lampen gebildete Plasma sendet das zur Sterilisation erforderliche UV-Licht aus. Beim zweiten Verfahren wird das Plasma zu Gas- oder Flüssigkeitsbehandlung eingesetzt. Beispiele für die Anwendung sind z. B., ein Ozongenerator für die Trinkwasserausbereitung oder PLexc prozessierter Luft (PPA).

Als mögliches Ausführungsbeispiel (siehe Figur 4), welches nicht als Beschränkung der Erfindung aufgefasst werden kann, wird ein Objekt 2 (wie z. B. Apfel) behandelt, dabei ist die Wirkung der reaktiven Spezies 21 auf das Objekt 2 indirekt und/oder semidirekt. Die reaktiven Spezies 21 wirken dabei in einem dünnen Kondensatfilm auch nach der aktiven Expositionsphase weiter. Die thermische Belastung des Objektes 2 bleibt gering, da auch bei kurzer Exposition mit einer aktiven Atmosphäre das Volumen des Objektes 2 kaum erwärmt wird. Wird z. B. ein dünner überwiegend wässriger Film auf der Oberfläche 2S der Objekts 2 abgeschieden, zeigt bereits ein Film von ca. 1 $\mu$m Dicke eine erhebliche Wirkung auf die chemischen Verbindungen und biologischen Spezies auf der Oberfläche 2S. Beispielsweise kann die gesamte Kondensationswärme, die auf 100 cm$^2$ der Oberfläche 2S des Objekts 2 freigesetzt wird, nur 20J betragen. Dadurch würde ein Objekt von 100 g Gewicht nur um ca. 0.1 K erwärmt werden.

Allerdings kann die Oberfläche 2S des Objekts 2 im Moment der Kondensation bei einer Schichtdicke von bis zu 1$\mu$m einen Temperatursprung bis zu der in der Kontaktkammer 10 herrschenden Taupunkttemperatur erfahren. Während dieser Kondensationsprozesse wirken alle reaktiven Spezies 21 besonders effektiv mit der Oberfläche 2S.

**Figur 6** zeigt eine schematische Darstellung einer möglichen Ausführungsform der Anordnung zur Behandlung von Objekten 2 mit reaktiven Spezies 21, die durch ein Plasma 20 angeregt wurden. Das Plasma 20 ist vorzugsweise ein atmosphärisches Kaltplasma, Nichtgleichgewichtsplasma oder DBE (dielektrisch behinderte Entladung). Die Plasmaleistung liegt zwischen 10W und 1000W, vorzugsweise bei 100W. Die thermische Leistung liegt zwischen 10W und 1000W, vorzugsweise bei 100W.

In einer Kontaktkammer 10 ist ein Plasmaerzeuger 1 (Plasmareaktor) vorgesehen, mittels dem die Erzeugung eines Plasmas 20 und/oder reaktiver Spezies 21 durchgeführt werden kann. Die Zusammensetzung der reaktiven Spezies 21 richtet sich nach dem zu behandelnden Objekt 2. Das zu behandelnde Objekt 2 ist bei der hier dargestellten Ausführungsform auf einer Bewegungseinrichtung 3 positioniert. Die Bewegungseinrichtung 3 kann verschiedene mögliche Ausgestaltungen annehmen, wie z. B. einen Drehteller, eine linearer Bewegungsmechanismus oder ein 3-dimnsionaler Bewegungsmechanismus. Die Bewegungseinrichtung 3 wird über eine Antriebseinheit 4 in geeigneter Weise bewegt, damit möglichst die gesamte Oberfläche 2S des Objekts 2 für die reaktiven Spezies 21 zugänglich ist. Eine Steuereinheit 5 ist mit mehreren Elementen, die für die Einstellung und/oder Einhaltung der Bedingungen in der Kontaktkammer 10 notwendig sind, verbunden. Zu den Elementen zählen eine Dosiereinheit 6, ein Zirkulationsgebläse 16, eine weitere Dosiereinheit 14, der Plasmaerzeuger 1, eine Energiequelle 51 des Plasmaerzeugers 1 (nicht dargestellt), mindestens ein Sensor 15, die Antriebseinheit 4, eine Heizung/Kühlung 9, eine Filtereinheit 8, die zur Umgebung 12 führt, sowie eine Türschleuse 7, die zur Umgebung 12 führt. Die Dosiereinheit 6 ist mit dem Plasmaerzeuger 1 über dessen mindestens einen Eingang 1E verbunden, so dass die Substanzen, wie z. B. Luft, Wasser und/oder weitere aktive Zusätze zugeführt werden können. Die aktiven Zusätze bestehen aus organischen Säuren, Alkoholen und Wasserstoffperoxid. Die den Plasmaerzeuger 1 zugeordnete Energiequelle 51 dient zur Stabilisierung der Temperatur im Plasmaerzeuger 1, was durch Zuheizen oder Kühlen erfolgen kann.

Eine Dosiereinheit 14, die in Form eines Dispensorventils ausgebildet ist, ist dem Plasmaerzeuger 1 zugeordnet, so dass damit ein Dispensieren der im Plasmaerzeuger 1 erzeugten reaktiven Spezies 21 in die Kontaktkammer 10 erfolgt. Die Bewegungseinrichtung 3 dient zur gleichmäßigen Exposition des Objektes 2 mit reaktiven Spezies 21. Es erfolgt eine Abscheidung eines reaktiven Kondensatfilms 13 aus reaktiven Spezies 21 auf der Oberfläche 2S des Objektes 2. Über einen Kondensatabscheider 11 erfolgt die Abscheidung des überschüssigen Kondensates aus der Kontaktkammer 10. Die Belüftung bzw. die geregelte Belüftung der Kontaktkammer 10 erfolgt über eine Filtereinheit 8 (Filtersystem). Die Filtereinheit 8 ist aus Aktivkohle aufgebaut oder umfasst katalytisch aktives Material zum Abbau von reaktiven Spezies 21 oder Nebenprodukten, wie z. B. Ozon und Stickoxide und Wasserstoffperoxid.

Für die Trocknung des Objektes 2 ist z. B. der Türschleuse 7 eine weitere der Heizung/Kühlung 9 zugeordnet. Die Türschleuse 7 ist mit einer Sperrvorrichtung (nicht dargestellt) versehen, um während der aktiven Exposition des Objekts 2 mit den reaktiven Spezies 21 eine Öffnung zu blockieren.

Der mindestens eine Sensor 15 dient zur Überwachung des Innenraums (Temperatur, Feuchte, Gaszusammensetzung). Das Zirkulationsgebläse 16 dient zur Durchmischung der Gasphase in der Kontaktkammer 10. Die Steuereinheit 5 dient zur Prozessregelung und Prozessüberwachung und ist hierzu kommunikativ mit Bewegungseinrichtung 3, der Antriebseinheit 4, der Dosiereinheit 6, der Energiequelle 51 des Plasmaerzeugers 1, dem Dispenserventil 14, dem Zirkulationsgebläse 16, dem mindestens einen Sensor 15, der Heizung/Kühlung 19 der Türschleuse 7 und der Türschleuse 7 verbunden.

In der Kontaktkammer 10 können abrupte Änderungen des pH-Wertes an der Oberfläche des Objekts 2, kurzzeitige Temperatursprünge an der Oberfläche 2S des Objekts 2, die Ausprägung eines reaktiven (oxidativen) Kondensatfilms 13 auf der Oberfläche 2S des Objekts 2 und die Exposition des Objekts 2 mit reaktiven Sauerstoffspezies überwacht werden.

Die Anwendung der erfindungsgemäßen Anordnung und des erfindungsgemäßen Verfahrens erfolgt im Wesentlichen zur Dekontamination von Lebensmitteln.

Die Erfindung wurde in Bezug auf bevorzugte Ausführungsformen beschrieben. Es ist für einen Fachmann jedoch selbstverständlich, dass Änderungen und Abwandlungen gemacht werden können, ohne dabei den Schutzbereich der nachstehenden Schutzansprüche zu verlassen.

**Bezugszeichenliste**

1 Plasmaerzeuger
1A Ausgang
1E Eingang
2 Objekt
2S Oberfläche des Objekts
3 Bewegungseinrichtung
4 Antriebseinheit
5 Steuereinheit
6 Dosiereinheit
7 Türschleuse
8 Filtereinheit
9 Heizung/Kühlung
10 Kontaktammer
11 Kondensatabscheider
12 Umgebung
13 Kondensatfilm
14 weitere Dosiereinheit
15 Sensor
16 Zirkulationsgebläse
18 Auflage
20 Plasma
21 reaktive Spezies
50 Anordnung
51 Energiequelle
52 Kontaktbereich
A Abstand

**Patentansprüche**

1. Anordnung zur Dekontamination einer Oberflächen (2S) von mindestens einem Objekt (2), umfassend einen Plasmaerzeuger (1), dem mindestens ein Eingang (1E), ein Ausgang (1A) und eine Energiequelle (51) zugeordnet ist, **dadurch gekennzeichnet**:

   • eine Kontaktkammer (10), die zumindest mit einer Dosiereinheit (14) des Plasmaerzeugers (1) fluide verbunden ist;
   • eine Dosiereinheit (6), die mit dem Plasmaerzeuger (1) über den mindestens einen Eingang (1E) fluide verbunden ist, so dass dem Plasmaerzeuger (1) Substanzen zur Erzeugung von reaktiven Spezies (21) zuführbar sind; und
   • eine Auflage (18) in der Kontaktkammer (10) zur Aufnahme des Objekts (2), die gegenüber der Dosiereinheit (14) vorgesehen ist, sodass die aus der Dosiereinheit (14) gesteuerten austretenden reaktiven Spezies (21) einen gleichmäßigen Kondensatfilm (13) auf einer Oberfläche (2S) des Objekts (2) ausbilden.

2. Anordnung nach Anspruch 1, wobei die aus der Dosiereinheit (14) in die Kontaktkammer (10) eintretenden reaktiven Spezies (21) eine Taupunkttemperatur aufweisen, die größer ist als eine Objekttemperatur.

3. Anordnung nach einem der vorangehenden Ansprüche, wobei eine Bewegungseinrichtung (3) zu Aufnahme des Objekts (2), die in der Kontaktkammer (10) vorgesehen ist und das Objekt (2) derart bewegt, dass der gleichmäßige Kondensatfilm (13) aus reaktiven Spezies (21) auf der Oberfläche (2S) des Objekts (2) ausbildbar ist.

**4.** Anordnung nach einem der vorangehenden Ansprüche, wobei ein Abstand (A) zwischen dem Objekt (2) und der Dosiereinheit (14) einstellbar ist, um das auf der Auflage (18) positionierte und zu behandelnde Objekt (2) in Bezug auf die Dosiereinheit (14) zu positionieren.

**5.** Anordnung nach einem der vorangehenden Ansprüche, wobei der Plasmaerzeuger (1) außerhalb der Kontaktkammer (10) vorgesehen ist.

**6.** Verfahren zur Dekontamination von Oberflächen von Objekten (2) mit reaktiven Spezies (21), **gekennzeichnet durch** die folgenden Schritte:

  • dass in einer Kontaktkammer (10) mindestens ein Objekt (2) auf einer Auflage (18) positioniert wird;
  • dass einem Plasmareaktor (1) von einer Dosiereinheit (6) Substanzen zugeführt werden, wobei im Plasmareaktor (1) daraus reaktive Spezies (21) gebildet werden;
  • dass über eine weitere, dem Plasmareaktor (1) zugeordnete, Dosiereinheit (14) die reaktiven Spezies (21) in die Kontaktkammer (10) verbracht werden, wobei über die Dosiereinheit (14) eine Eingangsmischung und die Taupunkttemperatur eingestellt werden; und
  • dass die Taupunkttemperatur der reaktiven Spezies (21) am Übergang der Dosiereinheit (14) in die Kontaktkammer (10) derart eingestellt wird, dass die Taupunkttemperatur größer als eine Temperatur der Objekts (2) ist, so dass ein gleichmäßiger Kondensatfilm (13) auf der Oberfläche (2S) des Objekts (2) ausgebildet wird.

**7.** Verfahren nach Anspruch 6, wobei die Auflage (18) eine Bewegungseinrichtung (3) ist, mit der das mindestens eine zu behandelnde Objekt (2) während der Behandlungsdauer mit den reaktive Spezies (21) bewegt wird, um den gleichmäßigen Kondensatfilm (13) der reaktiven Spezies (21) auf der Oberfläche (2S) des Objekts (2) auszubilden.

**8.** Verfahren nach einem der vorangehenden Ansprüche 6 bis 7, wobei dem Plasmaerzeuger (1) eine Energiequelle (51) zugeordnet ist, mit der eine Stabilisierung der Temperatur im Plasmaerzeuger (1) durch Zuheizen oder Kühlen erzielt wird.

**9.** Verfahren nach einem der vorangehenden Ansprüche 6 bis 8, wobei nach der Behandlung des Objekts (2) mit den reaktiven Spezies (21) das Objekt (2) zur Trocknung in eine Türschleuse (7) verbracht wird, der eine weitere Heizung/Kühlung (9), mit der die Temperatur für die Trocknung in der Türschleuse (7) eingestellt werden kann.

**10.** Verfahren nach einer der vorangehenden Ansprüche 6 bis 9, wobei eine Steuereinheit (5) mit mehreren Elementen für die Einstellung und/oder Einhaltung der Bedingungen in der Kontaktkammer (10) kommunikativ verbunden wird, wobei die Elemente die Dosiereinheit (6), ein Zirkulationsgebläse (16), die weitere Dosiereinheit (14), den Plasmaerzeuger (1), die Energiequelle (51) des Plasmaerzeugers (1), mindestens einen Sensor (15), die Antriebseinheit (4), die Heizung/Kühlung (9), eine Filtereinheit (8) sowie eine Türschleuse (7) umfassen.

Gerüche

Farbstoffe

Mikroorganismen

Hormone

Pestizide

**Fig. 1**

10

20, 21

20, 21

1

12

2

2

2S

2S

**Fig. 2**

**Fig. 3**

52

13

2S

2

18

**Fig. 4**

1E

1

1A

14

51

**Fig. 5**

Fig. 6

EP 3 732 992 A1

**Europäisches Patentamt**
**European Patent Office**
**Office européen des brevets**

# EUROPÄISCHER RECHERCHENBERICHT

Nummer der Anmeldung

EP 20 16 0321

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,P | WO 2020/003039 A1 (RELYON PLASMA GMBH [DE]) 2. Januar 2020 (2020-01-02) * das ganze Dokument *<br>----- | 1-10 | INV.<br>A23L3/26<br>A61L2/14<br>A61L2/20 |
| X | WO 2011/138463 A1 (INP GREIFSWALD LEIBNIZ INST FUER PLASMAFORSCHUNG UND TECHNOLOGIE E V []) 10. November 2011 (2011-11-10) * Absatz [0046] - Absatz [0054]; Abbildung 2 *<br>----- | 1,2,4-6, 8,10 | |
| X | WO 2017/218832 A1 (STERIFRE MEDICAL INC [US]) 21. Dezember 2017 (2017-12-21) * das ganze Dokument *<br>----- | 1-10 | |
| X | WO 2015/032888 A1 (KRÖMKER WILFRIED [DE]; INP GREIFSWALD E V [DE]) 12. März 2015 (2015-03-12) * Seite 8, Zeile 3 - Seite 13, Zeile 22; Abbildungen 1-5 *<br>----- | 1,2,4-6, 8-10 | |
| X | WO 2014/135254 A1 (AL KO THERM GMBH [DE]) 12. September 2014 (2014-09-12) * das ganze Dokument *<br>----- | 1,2,4-6, 10 | RECHERCHIERTE SACHGEBIETE (IPC)<br><br>A23L<br>A61L |
| X | EP 1 378 250 A1 (ETHICON INC [US]) 7. Januar 2004 (2004-01-07) * das ganze Dokument *<br>----- | 1,2,4-6, 10 | |
| X | WO 02/07788 A1 (PROTIC JACQUES [GB]) 31. Januar 2002 (2002-01-31) * das ganze Dokument *<br>----- | 1,2,4-6, 10 | |

Der vorliegende Recherchenbericht wurde für alle Patentansprüche erstellt

| Recherchenort | Abschlußdatum der Recherche | Prüfer |
|---|---|---|
| München | 24. September 2020 | Marti, Pedro |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 20 16 0321

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

24-09-2020

| Im Recherchenbericht angeführtes Patentdokument | Datum der Veröffentlichung | Mitglied(er) der Patentfamilie | Datum der Veröffentlichung |
|---|---|---|---|
| WO 2020003039 A1 | 02-01-2020 | DE 102018115300 A1<br>WO 2020003039 A1 | 02-01-2020<br>02-01-2020 |
| WO 2011138463 A1 | 10-11-2011 | EP 2566524 A1<br>JP 2013537433 A<br>US 2013142694 A1<br>WO 2011138463 A1 | 13-03-2013<br>03-10-2013<br>06-06-2013<br>10-11-2011 |
| WO 2017218832 A1 | 21-12-2017 | BR 112018076219 A2<br>CN 109862920 A<br>EP 3471782 A1<br>JP 2019523038 A<br>US 2019314535 A1<br>WO 2017218832 A1 | 18-06-2019<br>07-06-2019<br>24-04-2019<br>22-08-2019<br>17-10-2019<br>21-12-2017 |
| WO 2015032888 A1 | 12-03-2015 | CA 2923337 A1<br>DE 102013109777 A1<br>DE 202013012156 U1<br>DK 3041518 T3<br>EP 3041518 A1<br>RU 2016107819 A<br>TR 201802272 T4<br>US 2016220714 A1<br>WO 2015032888 A1 | 12-03-2015<br>12-03-2015<br>30-06-2015<br>26-02-2018<br>13-07-2016<br>12-10-2017<br>21-03-2018<br>04-08-2016<br>12-03-2015 |
| WO 2014135254 A1 | 12-09-2014 | CN 105025934 A<br>DE 102013003865 A1<br>EP 2964273 A1<br>KR 20150126612 A<br>WO 2014135254 A1 | 04-11-2015<br>11-09-2014<br>13-01-2016<br>12-11-2015<br>12-09-2014 |
| EP 1378250 A1 | 07-01-2004 | AU 2003205010 A1<br>CA 2433978 A1<br>DE 60307553 T2<br>EP 1378250 A1<br>ES 2269924 T3<br>JP 4322574 B2<br>JP 2004130082 A<br>US 2008025869 A1 | 15-01-2004<br>28-12-2003<br>20-09-2007<br>07-01-2004<br>01-04-2007<br>02-09-2009<br>30-04-2004<br>31-01-2008 |
| WO 0207788 A1 | 31-01-2002 | AU 2453002 A<br>EP 1303314 A1<br>US 2004022673 A1<br>WO 0207788 A1 | 05-02-2002<br>23-04-2003<br>05-02-2004<br>31-01-2002 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102008037898 A1
- DE 10036550 A1
- DE 102015119369 A1
- EP 3085244 A1
- US 2017112157 A1
- DE 102014213799 A1
- DE 102005061247 A1
- DE 102009025864 A1
- DE 102015204753 A1